# EUROPEAN PATENT SPECIFICATION

(11) **EP 3 569 142 B1**
(45) Date of publication and mention of the grant of the patent: **26.06.2024**
(21) Application number: 19173864.0
(22) Date of filing: 10.05.2019
(51) Int. Cl.: A61B 5/021, A61B 5/024, A61B 5/00

(54) **ELECTRONIC DEVICE FOR MEASURING BLOOD PRESSURE AND OPERATING METHOD THEREOF**
ELEKTRONISCHE VORRICHTUNG ZUR MESSUNG DES BLUTDRUCKS UND BETRIEBSVERFAHREN DAFÜR
DISPOSITIF ÉLECTRONIQUE POUR MESURER LA PRESSION ARTÉRIELLE ET SON PROCÉDÉ DE FONCTIONNEMENT

(30) Priority: 16.05.2018 KR 20180056014
(43) Date of publication of application: 20.11.2019
(73) Proprietor: Samsung Electronics Co., Ltd., Gyeonggi-do 16677 (KR)
(72) Inventor: Jeon, Taehan, 16677 Gyeonggi-do (KR); Lee, Hongji, 16677 Gyeonggi-do (KR); Park, Jongin, 16677 Gyeonggi-do (KR); Shin, Seunghwan, 16677 Gyeonggi-do (KR); Shim, Hwan, 16677 Gyeonggi-do (KR)
(74) Representative: Nederlandsch Octrooibureau

(56) References cited:
- WO-A1-2018/072175
- KR-A- 20180 024 266
- US-A1- 2016 022 166
- US-A1- 2017 109 495

## Description

### BACKGROUND

### 1. Field

The disclosure relates to an electronic device for measuring blood pressure and a control method thereof. More particularly, the disclosure relates to an apparatus and a method for reducing time required to measure blood pressure in an electronic device.

### 2. Description of Related Art

An electronic device may collect information related to health by using a biometric sensor. For example, information related to health may include blood pressure, blood glucose, a heart rate, an electrocardiogram, breathing, stress, oxygen saturation or the like. WO 2018/072175 A1 and US 2017/109495 A1 are examples of related art.

The above information is presented as background information only to assist with an understanding of the disclosure. No determination has been made, and no assertion is made, as to whether any of the above might be applicable as prior art with regard to the disclosure.

### SUMMARY

An electronic device may measure blood pressure by obtaining information related to person's heart rate by using one or more biometric sensors. To measure blood pressure, a method of using a pulse wave, such as pulse wave velocity (PWV) or pulse wave analysis (PWA) may be used. The PWV method and the PWA method include a process of generating a representative waveform by using a waveform of a photoplethysmogram (PPG) signal obtained from the biometric sensor. The electronic device may generate a representative waveform from which a feature point corresponding to a change in user's body can be distinctly analyzed, by overlaying a plurality of waveforms obtained from the PPG signal.

However, much time is required to generate the representative waveform, and as a result, much time is required to complete measurement of blood pressure.

Therefore, a need exists for an apparatus and a method for reducing time required to measure blood pressure in an electronic device.

Aspects of the disclosure are to address at least the above-mentioned problems and/or disadvantages and to provide at least the advantages described below. Accordingly, an aspect of the disclosure is to provide an apparatus and a method for reducing time required to measure blood pressure in an electronic device.

Another aspect of the disclosure is to provide an apparatus and a method for reducing time required to measure blood pressure by selecting one waveform template from a plurality of waveform templates based on at least one of a user's state, a user's posture, or a measuring environment, and by determining a blood pressure value of the user by using the selected waveform template.

Additional aspects will be set forth in part in the description which follows and, in part, will be apparent from the description, or may be learned by practice of the presented embodiments.

In accordance with an aspect of the disclosure, an electronic device is provided. The electronic device includes at least one sensor, a processor, and a memory operatively connected with the processor, and, when being executed, the memory may stores instructions that cause the processor to select one waveform template from a plurality of waveform templates stored in the memory, based on at least one of a state of a user, a posture of the user, or a measuring environment, and to determine a blood pressure value of the user by using the selected waveform template and a biometric signal obtained from the at least one sensor.

In accordance with another aspect of the disclosure, an operating method of an electronic device is provided. The method includes obtaining information related to at least one of a state of a user, a posture of the user, or a measuring environment, selecting one waveform template from a plurality of waveform templates stored in the electronic device, based on the obtained information, and determining a blood pressure value of the user by using the selected waveform template and a measured biometric signal.

Other aspects, advantages, and salient features of the disclosure will become apparent to those skilled in the art from the following detailed description, which, taken in conjunction with the annexed drawings, discloses various embodiments of the disclosure. The invention is set out in the appended claims.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1A is a view illustrating a shape of an electronic device according to an embodiment of the disclosure;
FIG. 1B is a view illustrating a shape of an electronic device according to an embodiment of the disclosure;
FIG. 2 is a schematic block diagram of an electronic device according to an embodiment of the disclosure;
FIG. 3A is a view illustrating a waveform template according to an embodiment of the disclosure;
FIG. 3B is a view illustrating a waveform template according to an embodiment of the disclosure;
FIG. 4A is a view illustrating operations of generating and analyzing a representative waveform according to an embodiment of the disclosure;
FIG. 4B is a view illustrating operations of generating and analyzing a representative waveform according to various embodiments of the disclosure;
FIG. 4C is a view illustrating operations of generating and analyzing a representative waveform according to an embodiment of the disclosure;
FIG. 5 is a flowchart for determining a blood pressure value in an electronic device according to an embodiment of the disclosure;
FIG. 6A is a flowchart for determining a blood pressure value based on a waveform template in an electronic device according to an embodiment of the disclosure;
FIG. 6B is a view illustrating an operation of measuring PPG similarity according to an embodiment of the disclosure;
FIGS. 7A, 7B, and 7C are views illustrating operations of generating a waveform template according to an embodiment of the disclosure;
FIG. 8A is a flowchart for determining a blood pressure value based on a waveform template in an electronic device according to an embodiment of the disclosure;
FIG. 8B is a view illustrating an operation of measuring similarity of a comparison waveform according to an embodiment of the disclosure;
FIG. 9 is a flowchart for determining a blood pressure value in an electronic device according to an embodiment of the disclosure;
FIGS. 10A, 10B, 10C, 10D, and 10E are views illustrating a screen of an electronic device which performs an operation of determining a blood pressure value according to various embodiments of the disclosure; and
FIG. 11 is a block diagram of an electronic device in a network environment according to an embodiment of the disclosure.

Throughout the drawings, like reference numerals will be understood to refer to like parts, components, and structures.

### DETAILED DESCRIPTION

The following description with reference to the accompanying drawings is provided to assist in a comprehensive understanding of various embodiments of the disclosure as defined by the claims. It includes various specific details to assist in that understanding but these are to be regarded as merely exemplary. In addition, descriptions of well-known functions and constructions may be omitted for clarity and conciseness. The terms which will be described below are terms defined based on the functions in the disclosure, and may be different according to users, intentions of the users, or customs. Therefore, the definitions of the terms should be made based on the contents throughout the specification.

The terms and words used in the following description and claims are not limited to the bibliographical meanings, but, are merely used by the inventor to enable a clear and consistent understanding of the disclosure. Accordingly, it should be apparent to those skilled in the art that the following description of various embodiments of the disclosure is provided for illustration purpose only and not for the purpose of limiting the disclosure as defined by the appended claims.

It is to be understood that the singular forms "a," "an," and "the" include plural referents unless the context clearly dictates otherwise. Thus, for example, reference to "a component surface" includes reference to one or more of such surfaces.

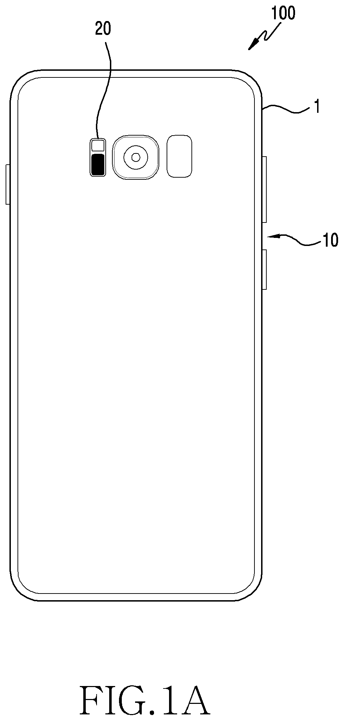

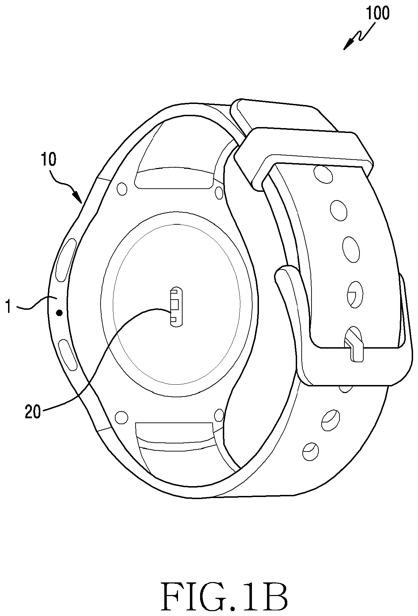

FIG. 1A is a view illustrating a shape of an electronic device according to an embodiment of the disclosure, and FIG. 1B is a view illustrating a shape of an electronic device according to an embodiment of the disclosure.

Referring to FIGS. 1A and 1B, an electronic device 100 may include a housing 1, a display 10, and a biometric sensor 20.

According to various embodiments of the disclosure, the housing 1 may provide a space for mounting elements (for example, the display 10, the biometric sensor 20, or the like) of the electronic device 100. The housing 1 may be implemented in various forms. According to an embodiment of the disclosure, as shown in FIG. 1, the housing 1 may be implemented in a rectangular shape having a curved surface for gripping by a user. However, this is merely an example, and various embodiments of the disclosure are not limited thereto. For example, the housing 1 may be implemented in various shapes, such as a circle, a square, an oval, or the like so as to allow a user to grip the electronic device 100. According to another embodiment of the disclosure, as shown in FIG. 1B, the housing 1 may be implemented in a circular shape to be attachable to a part of user's body, but various embodiments of the disclosure are not limited thereto. For example, the housing 1 may be implemented in various shapes, such as a rectangle, a square, an oval, or the like to be attachable to a part of the user's body.

According to various embodiments of the disclosure, the display 10 may be used to provide information processed in the electronic device 100. The display 10 may display a screen or a user interface regarding information processed in the electronic device 100. According to various embodiments of the disclosure, the display 10 may be disposed on a front surface of the housing 1 to provide information processed in the electronic device 100. The display 10 may be exposed through a part of the front surface of the housing 1 to provide information processed in the electronic device 100.

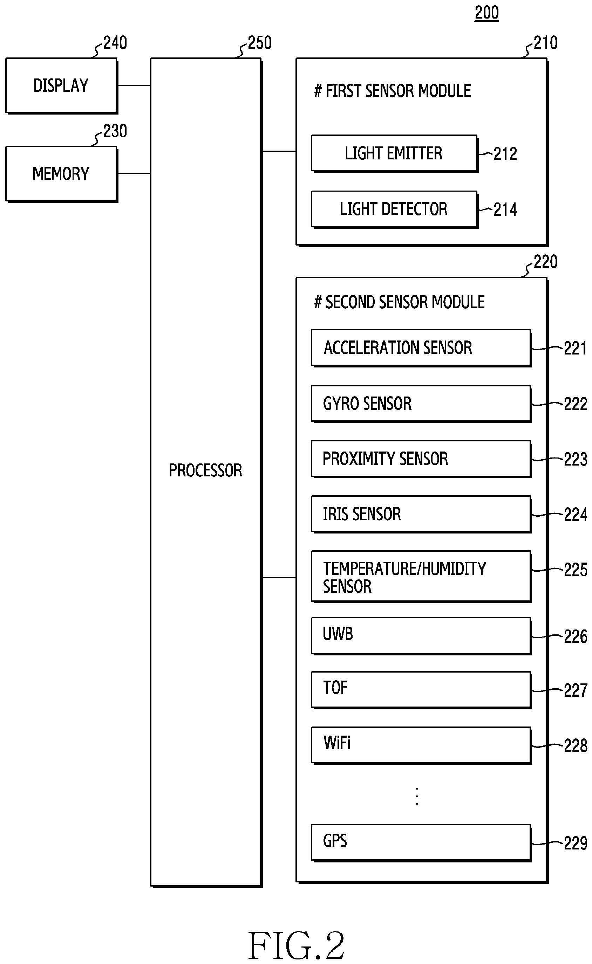

According to various embodiments of the disclosure, the biometric sensor 20 may be used to obtain biometric information of the user of the electronic device 100. According to various embodiments of the disclosure, the biometric sensor 20 may emit light toward a part of the user's body contacting the electronic device 100, by using a light emitter formed of one or more light emitting elements, as will be described below through FIG. 2. In addition, the biometric sensor 20 may receive reflected light with respect to the emitted light by using a light detector, such as a photodiode or the like. The biometric sensor 20 may convert information regarding the reflected light into an electric signal. The electric signal obtained through the biometric sensor 20 may be transmitted to a processor in the electronic device 100. The transmitted electric signal may include information regarding biometric information of the user.

According to various embodiments of the disclosure, the biometric sensor 20 may be disposed on the rear surface or front surface of the housing 1 to measure biometric information of the user. The rear surface may be a surface that is disposed in the opposite direction to the front surface. The biometric sensor 20 may be exposed through a part of the rear surface or the front surface of the housing 1 to measure biometric information of the user. For example, as shown in FIG. 1A, the biometric sensor 20 may be disposed on an upper end of the rear surface of the housing 1 to allow the user to bring a part of user's body (for example, a finger or the like) into contact with the biometric sensor 20 while gripping the electronic device 100 (or the housing 1). In another example, as shown in FIG. 1B, the biometric sensor 20 may be disposed on a center of the rear surface of the housing 1 to allow the user to bring a part of user's body (for example, a skin surface close to radial arteries or the like) into contact with the biometric sensor 20 while wearing the electronic device 100 (or the housing 1). The positions of the biometric sensor 20 illustrated in FIGS. 1A and 1B are merely examples, and various embodiments of the disclosure are not limited thereto. For example, the biometric sensor 20 may be disposed on various positions of the electronic device 100 to allow a part of user's body to be in contact therewith.

According to various embodiments of the disclosure, the electronic device 100 may further include a film (not shown) attached to, disposed on (over), superimposed on (over), or overlaid on (over) the biometric sensor 20. The film may be used to measure glucose, and may include chemochromic materials. The film may be configured with a translucent state or a transparent state to apply light of a necessary wavelength to the user. According to an embodiment of the disclosure, when the film is configured with the transparent state, the biometric sensor 20 may further include an element (for example, a light emitting diode) to emit light of a necessary wavelength.

FIG. 2 is a schematic block diagram of an electronic device according to an embodiment of the disclosure.

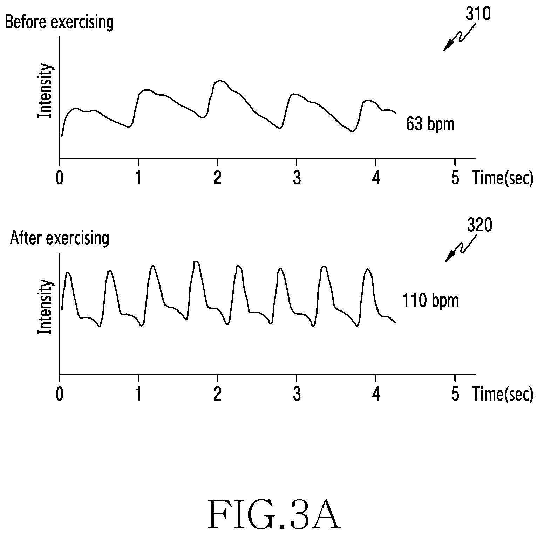

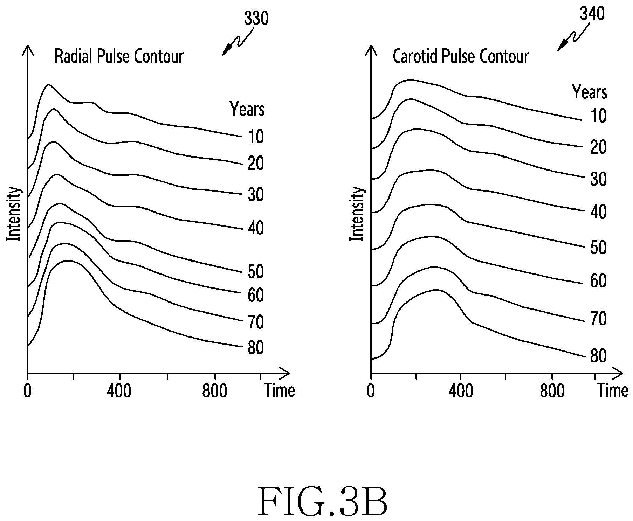

FIG. 3A is a view illustrating a waveform template according to an embodiment of the disclosure, FIG. 3B is a view illustrating a waveform template according to an embodiment of the disclosure.

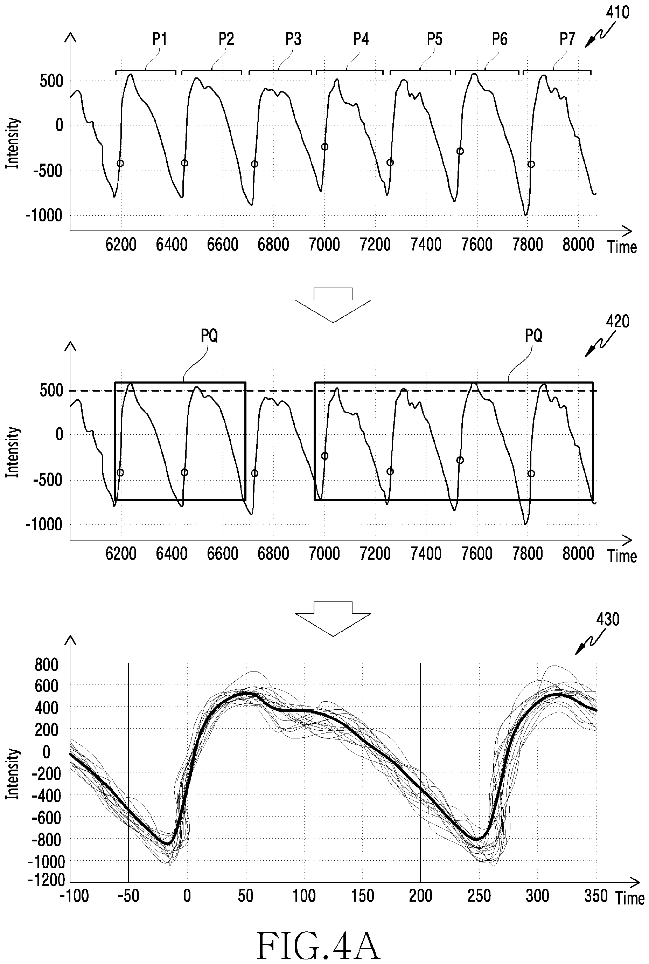

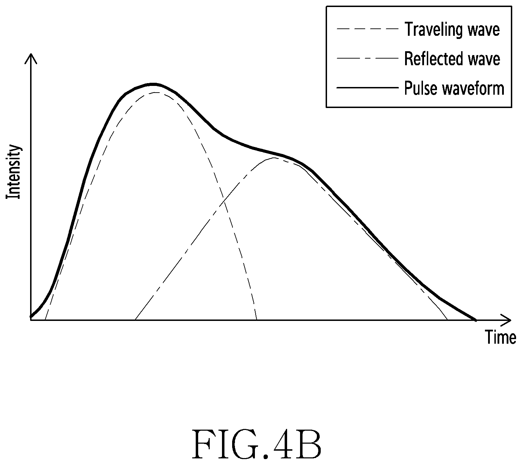

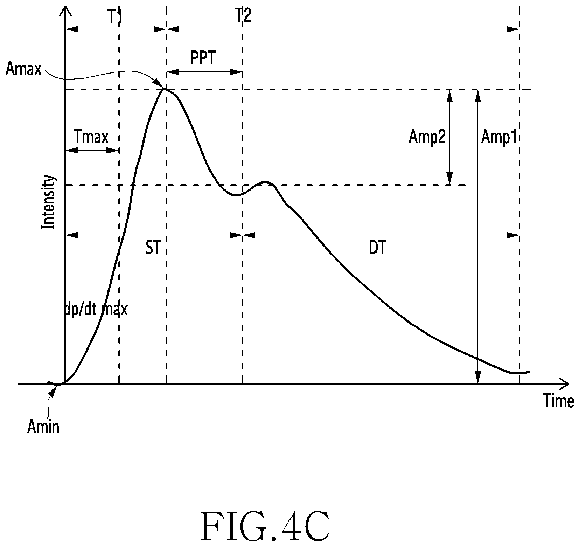

FIG. 4A is a view illustrating operations of generating and analyzing a representative waveform according to an embodiment of the disclosure, FIG. 4B is a view illustrating operations of generating and analyzing a representative waveform according to an embodiment of the disclosure, and FIG. 4C is a view illustrating operations of generating and analyzing a representative waveform according to an embodiment of the disclosure. An electronic device 200 of FIG. 2 may be the electronic device 100 of FIGS. 1A and 1B.

Referring to FIG. 2, the electronic device 200 may include a first sensor module 210, a second sensor module 220, a memory 230, a display 240, and a processor 250. However, this should not be considered as limiting, and the electronic device 200 may further include other elements, such as a camera module, an audio module, or the like.

According to various embodiments of the disclosure, the first sensor module 210 may include a biometric sensor (for example, the biometric sensor 20 of FIGS. 1A and 1B). For example, the first sensor module 210 may be configured with a light emitter 212 and a light detector 214, and may be used to directly or indirectly measure pulse wave information of a user. According to an embodiment of the disclosure, the first sensor module 210 may be used to obtain information related to a state of the user. The state of the user may include a body state (for example, a normal heart rate state, an abnormal heart rate state, or the like) or a psychological state (for example, a stable state, an unstable state, an impatient state).

According to various embodiments of the disclosure, the light emitter 212 may include a light-emitting diode (LED) having a plurality of wavelengths. For example, the plurality of wavelengths may include at least one green wavelength, red wavelength, blue wavelength, or infrared (IR) wavelength. However, this is merely an example, and the disclosure is not limited thereto. For example, the light emitter 212 may be configured with an LED having an IR wavelength and an LED having a red wavelength. According to various embodiments of the disclosure, the green wavelength may have the advantage of being robust to a noise signal since a change in the degree of scattering caused by the flow of bloodstream is well detected. The electronic device 200 may measure a heart rate by using the green wavelength. According to various embodiments of the disclosure, in the case of the red wavelength, an amount of light absorbed varies according to a blood flow rate. Therefore, the electronic device 200 may measure a heart rate by using the red wavelength. In addition, the electronic device 200 may obtain a variety of biometric information, such as oxygen saturation (SpO₂), in addition to the heart rate, by using the red wavelength and the IR wavelength altogether. According to various embodiments of the disclosure, the electronic device 200 may measure a skin tone by combining the red wavelength, the green wavelength, and the IR wavelength. The light emitter 212 may further include LEDs of various wavelength bands to measure more biometric information. For example, the electronic device 200 may measure a variety of biometric information by additionally using a light emitter 211 having a white wavelength.

According to various embodiments of the disclosure, the light detector 214 may include one or more photodiodes. The light detector 214 may receive light outputted from the light emitter 212 and reflected from an object (for example, the user). The light detector 214 may convert the received light into an electric signal. According to an embodiment of the disclosure, the light detector 214 may generate (or obtain) a photoplethysmogram (PPG) signal by using the received light. According to various embodiments of the disclosure, the light detector 214 may be disposed to be spaced apart from the light emitter 212 by a predetermined distance. However, this should not be considered as limiting, and a plurality of light detectors 214 may be configured and may be disposed to be spaced apart from the light emitter 212 by different distances, respectively. For example, the plurality of light detector 214 may have different absorption wavelength bands, and may obtain various kinds of biometric information by receiving light of a corresponding wavelength band.

According to various embodiments of the disclosure, the second sensor module 220 may be used to obtain information related to at least one of a posture of the electronic device 200 (or the user) or a measuring environment. According to an embodiment of the disclosure, the second sensor module 220 may include an acceleration sensor 221, a gyro sensor 222, and a proximity sensor 223, which obtain information related to the posture (or motion), movement, or the like of the electronic device 200. In addition, the second sensor module 220 may include an iris sensor 224, and a temperature sensor (not shown) which obtain information related to the user. According to an embodiment of the disclosure, the second sensor module 220 may include a temperature/humidity sensor 225, an ultra-wideband (UWB) sensor 226, an infrared laser distance measurement (time of flight (ToF)) sensor 227, an illuminance sensor (not shown), or the like, which obtain information related to the measuring environment (for example, a surrounding environment of the user). According to an embodiment of the disclosure, the second sensor module 220 may further include a communication module, such as WiFi 228, global positioning system (GPS) 229, or the like to recognize a situation or location of the electronic device 200. According to an embodiment of the disclosure, the second sensor module 220 may include at least one of a taste sensor (not shown) or an olfactory sensor (not shown) that can obtain information related to at least one of a kind of food, a taste of food, or an ingredient of food ingested by the user. The taste sensor or olfactory sensor may be disposed inside the electronic device 200 or may be implanted in a part of user's body (for example, a tooth, an artificial tooth). For example, information obtained through the second sensor module 220 implanted in a part of the user's body may be transmitted to the processor 250 of the electronic device 200. According to various embodiments of the disclosure, the second sensor module 220 and the first sensor module 210 may be the physically same sensor and may perform different functions. For example, at least part of the second sensor module 220, such as the acceleration sensor 221, the gyro sensor 222, the proximity sensor, or the like, may perform the function of the first sensor module 210.

According to various embodiments of the disclosure, the memory 230 may store at least one representative waveform used to measure biometric information (for example, blood pressure) of the user and/or information related to the representative waveform. The information related to the representative waveform may include at least one of blood pressure measured through the representative waveform, posture information of the user, state information of the user, or measuring environment information. The blood pressure measured through the representative waveform may include at least one of a systolic blood pressure value, a diastolic blood pressure value, or a mean arterial pressure value. The posture information of the user is information associated with the posture of the user (or the electronic device 200) when blood pressure is measured, and may include a state of the user during sleeping, a state of the user during resting, a state of the user during exercising, a state of the user after exercising, or the like. The state information of the user may include at least one of a body state or a psychological state of the user when blood pressure is measured, as described above. The measuring environment information may include at least one of location information of the user (or the electronic device 200), and temperature/humidity information when blood pressure is measured. In addition, the information related to the representative waveform may include at least one of information related to user's health, such as sex, age, height, and weight of the user.

Referring to FIGS. 3A and 3B, in the following description, at least one representative waveform and information related to the representative waveform will be referred to as a "waveform template." The waveform template according to various embodiments may be stored in the form as shown in FIGS. 3A and 3B. A representative waveform in a state without a motion 310 (for example, a state before exercising), and a representative waveform in a state with a motion 320 (for example, a state after exercising) will be described by way of an example. Such two types of representative waveforms may be distinguished from each other. For example, as shown in FIG. 3A, the waveform template associated with the state with the motion may include a representative waveform having high peaks in comparison to the waveform template associated with the state without the motion. In addition, each of the waveform templates may include at least one of a systolic blood pressure value, a diastolic blood pressure value, or a mean arterial pressure value which was previously measured by using the representative waveform included in the waveform template. For example, as shown in the drawings, a blood pressure value (for example, 110 mmHg) measured with the representative waveform in the state with the motion may be stored along with the waveform template, and a blood pressure value (for example, 63 mmHg) measured with the representative waveform in the state without the motion may be stored along with the waveform template. In another example, as shown in FIG. 3B, as user's age increases, the contractility of blood vessels is reduced, and the intensity of a subsequent peak of a radial pulse 330 and/or carotid pulse 340 is reduced due to reflected bloodstream. Accordingly, a waveform template having a different representative waveform according to user's age may be stored.

According to various embodiments of the disclosure, the display 240 may provide a variety of information to the user. According to an embodiment of the disclosure, the display 240 may display at least one of the systolic blood pressure value, the diastolic blood pressure value, or the mean arterial pressure value. The blood pressure value may be displayed in the form of a number, a graph, a table, or the like. According to another embodiment of the disclosure, the display 240 may display a guide message for measuring blood pressure. For example, the guide message may include guide information for guiding the first sensor module 210 of the electronic device 200 to be placed on the radial arteries of user's wrist.

According to various embodiments of the disclosure, the processor 250 may measure a blood pressure value by obtaining a variety of biometric information through the first sensor module 210. The blood pressure may be understood as the pressure of blood sent from the heart on the walls of blood vessels. The electronic device 200 according to an embodiment may measure a blood pressure value by using a representative waveform as in the PWV or PWA method. The representative waveform may be understood as one waveform obtained by overlaying a plurality of waveforms obtained from pulse waves one on another.

According to an embodiment of the disclosure, when a blood pressure measurement event occurs, the processor 250 may use a waveform template including a representative waveform used to measure blood pressure in the past, and information related to the representative waveform, in order to determine a user's blood pressure value. For example, the processor 250 may determine at least one of the state of the user, the posture of the user, or the measuring environment, based on at least one of the first sensor module 210 or the second sensor module 220. Based on this, the processor 250 may select one waveform template to be used to determine a blood pressure value from a plurality of waveform templates stored.

According to an embodiment of the disclosure, the processor 250 may determine a blood pressure value of the user by using the selected waveform template, and a waveform of a PPG signal obtained from the first sensor module 210. For example, when similarity between the waveform of the PPG signal and the selected waveform template falls within a specified range, the processor 250 may determine a blood pressure value stored in association with the selected waveform template (or a blood pressure value included in the waveform template), as a blood pressure value of the user. In another example, when the similarity between the waveform of the PPG signal and the selected waveform template falls within the specified range, a blood pressure value of the user may be determined by analyzing a feature point of the representative waveform of the waveform template. The above-described two methods omit an operation of generating a representative waveform by using a PPG signal when blood pressure is measured, and thus can reduce time required to measure blood pressure in the electronic device 200. According to various embodiments of the disclosure, the processor 250 may add the waveform of the PPG signal used to measure the similarity to the waveform template, after determining the blood pressure value.

Referring to FIGS. 4A, 4B, and 4C, according to an embodiment of the disclosure, when the similarity between the waveform of the PPG signal and the selected waveform template falls out of the specified range, the processor 250 may obtain (or generate) a representative waveform by processing the PPG signal obtained through the first sensor module 210, and may measure a blood pressure value of the user by using the representative waveform. For example, as shown in FIG. 4A, the processor 250 may obtain a plurality of waveforms P1 to P7 (for example, waveforms of the PPG signal) from which a noise is removed by amplifying or filtering the signal of the first sensor module 210 (410), may extract a plurality of waveforms PQs (for example, waveforms having peaks higher than or equal to a threshold value (for example, 500 mV)) that satisfy a condition from among the obtained plurality of waveforms (420), and may generate a representative waveform (430). The representative waveform may be interpreted as one waveform in which extracted waveforms (for example, 10 waveforms) satisfying the condition are overlaid one on another. In addition, as shown in FIG. 4B, a representative waveform may be configured by overlaying a traveling wave generated from the heart and traveling, and a reflected wave returning from a distal end part, and augmentation of a pulse wave may occur as the reflected wave is overlaid on the traveling wave. Since the shape of the representative waveform reflects a state of the cardiovascular system or blood pressure, the processor 250 may measure a blood pressure value of the user by extracting various feature points from the representative waveform. For example, the processor 250 may measure a cardiac output and/or total peripheral resistance (TPR) by analyzing the generated representative waveform, and may measure at least one of the systolic blood pressure value or the diastolic blood pressure value based on the measured cardiac output and/or TPR. In this case, as shown in FIG. 4C, the processor 250 may measure a blood pressure value by extracting (or analyzing), from the generated representative waveform, at least one of a time T1 required to reach the maximum peak, a duration time T2 from the maximum peak to the next waveform, a time PPT between the traveling wave and the reflected wave, a systole sustaining time (ST), a diastole sustaining time (DT), systolic blood pressure (Amax), diastolic blood pressure (Amin), a change in pressure per hour (dp/dt max), amplitude of the systole Amp1, and a difference between the amplitude of the systole and the amplitude of the diastole. In this case, the processor 250 may generate a new waveform template based on the representative waveform obtained by processing the PPG signal, and information related to the representative waveform (for example, at least one piece of information of state information of the user, posture information of the user, or environment information), and may store the new waveform template. As described above, the method of measuring the blood pressure value based on the representative waveform obtained by processing the signal is merely one embodiment of the disclosure, and various well-known technologies related to the measurement of a blood pressure value may be referred to as various embodiments of the disclosure.

According to an embodiment of the disclosure, the processor 250 may not select a waveform template to be used to determine a blood pressure value of the user. For example, a waveform template may not be stored in the electronic device 200 (for example, the memory 230). In addition, a waveform template may be stored in the electronic device 200, but the waveform template may not correspond to state information (for example, a heart rate, and the like) of the user, a posture (or motion) of the user, or environment information (for example, temperature, humidity, location, or the like). Even in this case, the processor 250 may measure a blood pressure value based on a representative waveform obtained by processing a PPG signal, and may store a waveform template including the representative waveform used to measure the blood pressure value, and information related to the representative waveform, as described above.

According to various embodiments of the disclosure, an electronic device (for example, the electronic device 200 of FIG. 2) may include at least one sensor (for example, the first sensor module 210, the second sensor module 220 of FIG. 2), a processor (for example, the processor 250 of FIG. 2), and a memory (for example, the memory 230 of FIG. 2) operatively connected with the processor, and, when being executed, the memory may store instructions that cause the processor to select one waveform template from a plurality of waveform templates stored in the memory, based on at least one of a state of a user, a posture of the user, or a measuring environment, and to determine a blood pressure value of the user by using the selected waveform template and a biometric signal obtained from the at least one sensor. According to an embodiment of the disclosure, the biometric signal may include a PPG signal.

According to various embodiments of the disclosure, the at least one sensor may include a biometric sensor (for example, the first sensor module 210) configured to obtain information related to the state of the user, and an additional sensor (for example, the second sensor module 220) configured to obtain information related to at least one of the posture of the user or the measuring environment. According to an embodiment of the disclosure, the information related to the measuring environment may include at least one of a location of the electronic device, a temperature or humidity when blood pressure is measured. According to an embodiment of the disclosure, the additional sensor may include a gesture sensor, a gyro sensor, an atmospheric pressure sensor, a magnetic sensor, an acceleration sensor, a grip sensor, a proximity sensor, a color sensor, an IR sensor, a temperature sensor, a humidity sensor, or an illuminance sensor.

According to various embodiments of the disclosure, the instructions may include an instruction that causes the processor to select one waveform template from the plurality of waveform templates, based on at least one of a heart rate of the user or environment information when blood pressure is measured.

According to various embodiments of the disclosure, the instructions may include an instruction that causes the processor to determine whether the selected waveform template is used, based on a similarity indicating how much the selected waveform template and the biometric signal are similar to each other.

According to various embodiments of the disclosure, the instructions may include an instruction that causes the processor to, when the similarity indicating how much the selected waveform template and the biometric signal are similar to each other falls within a specified similarity range, determine the blood pressure value of the user based on the selected waveform template.

According to various embodiments of the disclosure, the instructions may include an instruction that causes the processor to determine the blood pressure value of the user based on the selected waveform template, and then to add a waveform template including the biometric signal.

According to various embodiments of the disclosure, the instructions may include an instruction that causes the processor to, when the similarity indicating how much the selected waveform template and the biometric signal are similar to each other does not fall within the specified similarity range, generate a representative waveform by using the biometric signal, to measure the blood pressure value of the user based on the generated representative waveform, and to add a waveform template corresponding to the generated representative waveform.

According to various embodiments of the disclosure, the instructions may include an instruction that causes the processor to generate the representative waveform based on at least one of the state of the user, the posture of the user, or the measuring environment when adding the waveform template corresponding to the representative waveform, and to add the generated representative waveform to the waveform template.

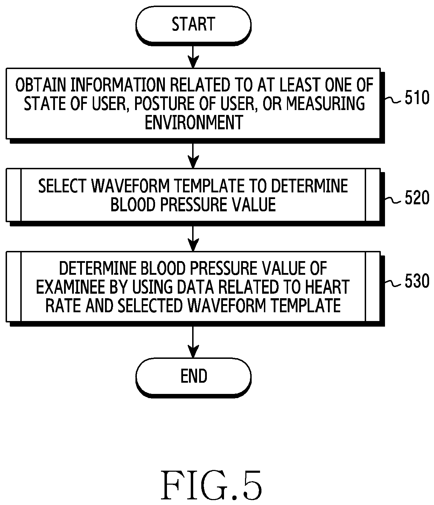

FIG. 5 is a flowchart for determining a blood pressure value in an electronic device according to an embodiment of the disclosure. In the following embodiments of the disclosure, respective operations may be performed in sequence, but may not necessarily be performed in sequence. For example, the order of the operations may be changed, or at least two operations may be performed in parallel. The electronic device of FIG. 5 may be the electronic device 200 of FIG. 2.

Referring to FIG. 5, the electronic device (for example, the processor 250 of FIG. 2) according to the invention obtains information related to at least one of a state of a user (for example, an examinee), a posture of the user, or a measuring environment in operation 510. According to the invention, the information related to at least one of the state of the user, the posture of the user, or the measuring environment may be obtained through at least one of a biometric sensor (for example, the first sensor module 210 of FIG. 2) or an additional sensor (for example, the second sensor module 220 of FIG. 2). According to the invention, the information related to at least one of the state of the user, the posture of the user, or the measuring environment is obtained in response to a part of the user's body (for example, a finger) contacting or approaching the biometric sensor being detected.

According to various embodiments of the disclosure, in operation 520, the electronic device (for example, the processor 250) may select a waveform template for determining a blood pressure value. For example, the processor 250 may select one waveform template from a plurality of waveform templates, based on at least one of the state of the user, the posture of the user, or the measuring environment. According to an embodiment of the disclosure, the heart rate of the user may be measured based on a PPG signal obtained through the biometric sensor (for example, the first sensor module 210 of FIG. 2), and the processor 250 may select a waveform template corresponding to a body state (for example, a normal heart rate state, an abnormal heart rate state, and the like) of the user, or a psychological state (for example, a stable state, an unstable state, an impatient state, and the like) of the user, based on the measured heart rate. According to another embodiment of the disclosure, the posture of the user (for example, a state in which the user measures blood pressure by using the left hand or right hand, a standing state, a sitting state, a dynamic state, a static state, a sleeping state, a resting state, an exercising state, or a state after exercising, and the like) may be determined based on motion information obtained through the additional sensor (for example, the second sensor module 220 of FIG. 2), and the processor 250 may select a waveform template corresponding to the determined posture of the user. According to still another embodiment of the disclosure, a measuring environment of biometric information may be determined based on the environment information (for example, location information, temperature information, or humidity information) obtained through the additional sensor, and the processor 250 may select a waveform template corresponding to the determined measuring environment.

According to various embodiments of the disclosure, in operation 530, the electronic device (for example, the processor 250) may determine a blood pressure value of the user by using data related to the heart rate and the selected waveform template. The data related to the heart rate may include the waveform of the PPG signal. According to an embodiment of the disclosure, the waveform template may include a representative waveform used to measure blood pressure in the past, and at least one of a systolic blood pressure value, a diastolic blood pressure value, or a mean arterial pressure value which was previously measured by using the representative waveform. Accordingly, when similarity between the waveform of the PPG signal and the waveform template falls within a pre-specified range, the processor 250 may determine a blood pressure value included in the waveform template as a blood pressure value of the user. In addition, when the similarity between the waveform of the PPG signal and the waveform template falls out of the pre-specified range, the processor 250 may generate a representative waveform by processing the PPG signal, and may measure a blood pressure value of the user based on the generated representative waveform. According to an embodiment of the disclosure, before generating the representative waveform by processing the PPG signal, the processor 250 may display guide information to guide the first sensor module 210 to be placed on a part to be examined (for example, the radial arteries of user's wrist) in order to increase reliability of the similarity.

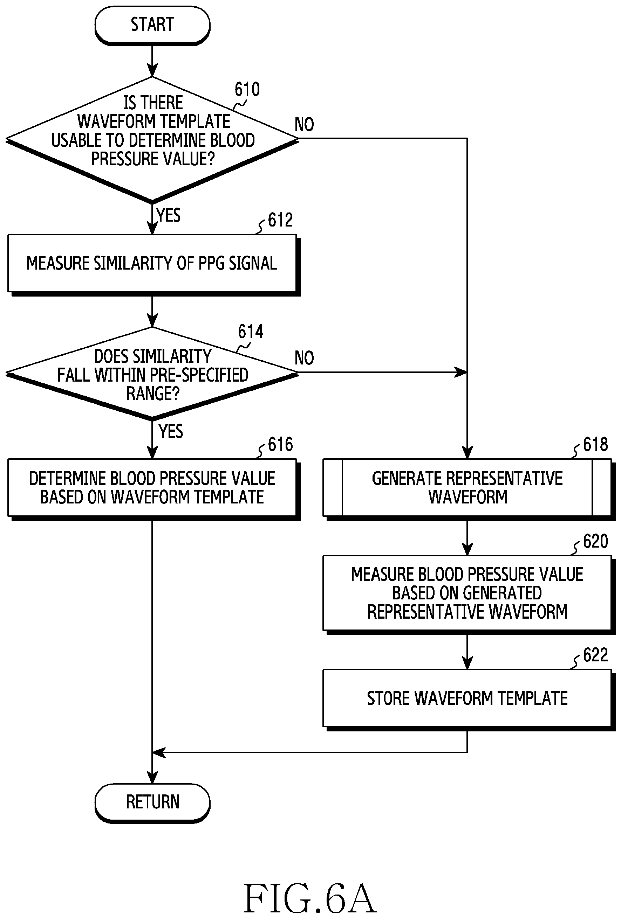

FIG. 6A is a flowchart for determining a blood pressure value based on a waveform template in an electronic device according to an embodiment of the disclosure.

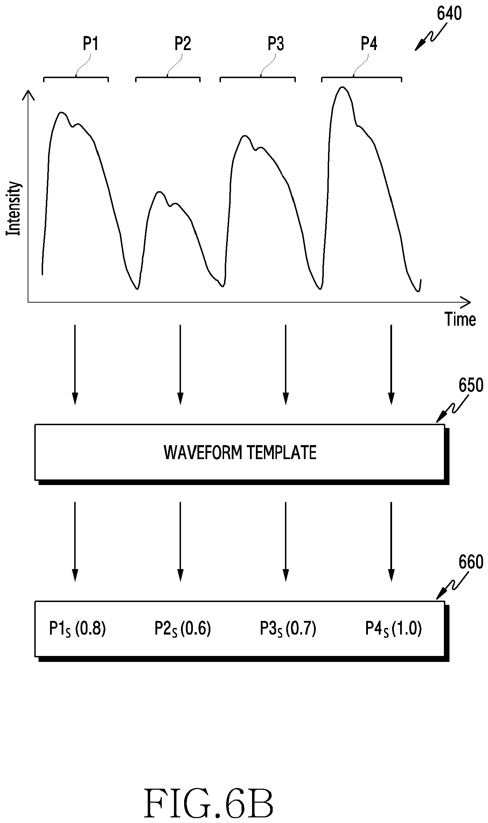

FIG. 6B is a view illustrating an operation of determining PPG similarity according to an embodiment of the disclosure.

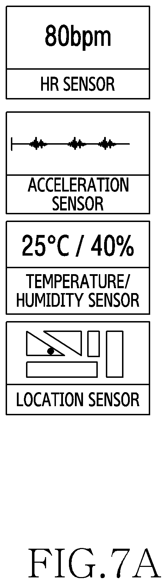

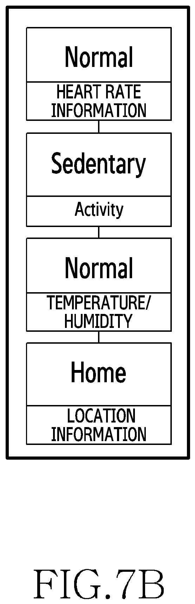

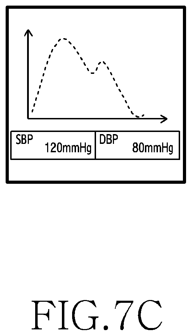

FIG. 7A is a view illustrating an operation of generating a waveform template according to an embodiment of the disclosure, FIG. 7B is a view illustrating an operation of generating a waveform template according to an embodiment of the disclosure, and FIG. 7C is a view illustrating an operation of generating a waveform template according to an embodiment of the disclosure.

Operations of FIG. 6A, which will be described below, indicate various embodiments of operations 520 and 530 of FIG. 5. In the following embodiments of the disclosure, respective operations may be performed in sequence, but may not necessarily be performed in sequence. For example, the order of the operations may be changed, or at least two operations may be performed in parallel. The electronic device of FIGS. 6A and 6B may be the electronic device 200 of FIG. 2.

Referring to FIG. 6A, the electronic device (for example, the processor 250 of FIG. 2) according to various embodiments may determine whether there exists a waveform template to be used to determine a blood pressure value in operation 610. The waveform template to be used to determine the blood pressure value may be associated with at least one of a body (or psychological) state of the user (for example, an examinee), a posture of the user, or environment information (for example, location information, temperature/humidity information, or the like). The existence of the waveform template to be used to determine the blood pressure value may mean that there is a history of having measured a blood pressure in the past in a condition similar to a current blood pressure measuring condition. According to various embodiments of the disclosure, the processor 250 may determine whether there exists a waveform template, based on whether there is the history of having measured blood pressure in the past. The waveform template may be generated and stored every time blood pressure is measured, and, when the blood pressure measuring operation is initially performed, a waveform template may not be stored in the electronic device (for example, the memory 230 of FIG. 2). For example, when the blood pressure measuring operation is initially performed, the processor 250 may determine that there does not exist a waveform template. According to various embodiments of the disclosure, the processor 250 may determine at least one of the body (psychological) state of the user, or the measuring environment (for example, location information, temperature information, or humidity information) based on a biometric sensor (for example, the first sensor module 210 of FIG. 2) or an additional sensor (for example, the second sensor module 220 of FIG. 2), and may determine whether there exists a waveform template based on the determined information. According to an embodiment of the disclosure, the processor 250 may determine whether there exists a waveform template including information corresponding to the determined user's state and/or environment, from among waveform templates stored. According to various embodiments of the disclosure, the processor 250 may determine whether there exists a waveform template, based on reliability regarding the stored waveform templates. The reliability may be associated with a generation time of the waveform template. For example, the processor 250 may determine that a waveform template which is a predetermined time (for example, one month) long since the waveform template was generated has low reliability. When there exists a waveform template corresponding to the state of the user and/or environment, but the reliability of the waveform template is low, the processor 250 may determine that there does not exist a waveform template to be used to determine a blood pressure value.

According to various embodiment of the disclosure, when it is determined that there does not exist a waveform template to be used to determine a blood pressure value, the electronic device (for example, the processor 250) may perform an operation of obtaining a representative waveform by processing a PPG signal in operation 618. For example, the processor 250 may generate the representative waveform by using the method described above through FIG. 4A. According to an embodiment of the disclosure, the processor 250 may generate the representative waveform by processing the PPG signal, and then, in operation 620, may measure a blood pressure value of the user by using the generated representative waveform. For example, the processor 250 may measure a blood pressure value of the user by analyzing at least one of feature points of the representative waveform described above through FIG. 4C. According to an embodiment of the disclosure, the processor 250 may measure the blood pressure value of the user, and then, may store a waveform template based on the generated representative waveform in operation 622. For example, the processor 250 may collect information associated with the body (psychological) state of the user, the posture of the user and/or the measuring environment by using at least one sensor of the first sensor module 210 or the second sensor module 220, and may use the collected information to generate the waveform template.

Referring to FIG. 7A, the processor 250 may collect heart rate information as the information associated with the body (or psychological) state of the user by using at least one sensor of the first sensor module 210 or the second sensor module 220, may collect at least one of the ambient temperature/humidity information of the electronic device, and the location information of the electronic device as the environment information, and may collect acceleration information as the posture information of the user.

Referring to FIG. 7B, the processor 250 may generate information associated with a representative waveform generated by using the collected information. For example, the processor 250 may determine information of a situation in which a blood pressure value is measured when the heart rate of the user is 80 bpm, the posture of the electronic device is a static posture, the ambient temperature/humidity of the electronic device is 25 °C/40%, and the user is positioned in the house, and may generate information associated with the representative waveform based on at least one piece of information of the above-mentioned information.

Referring to FIG. 7C, the processor 250 may generate the waveform template by storing the information generated as shown in FIG. 7B along with the representative waveform. The information measured at the second sensor module 220 may classify situation information of each sensor according to a predetermined criterion. For example, the processor 250 may classify the heart rate into a heart rate of a normal range (for example, 60-100 bpm), and a heart rate of the other range (for example, a high heart rate, a low heart rate, and the like), and may generate a waveform template by using the heart rate information. In this case, the processor 250 may classify a heart rate of a normal range according to sex or age, and may generate a waveform template by using the heart rate information. In another example, the processor 250 may classify the temperature/humidity into temperature/humidity of a normal range, and temperature/humidity of the other range (for example, high temperature/high humidity, low temperature/high humidity, low temperature/low humidity, and the like).

According to the invention, when it is determined that there exists a waveform template to be used to determine a blood pressure value, the electronic device (for example, the processor 250) measures similarity of the PPG signal in operation 612. The processor 250 measures similarity by comparing the PPG signal received for a pre-specified time, and the waveform template. For example, as shown in FIG. 6B, the processor 250 may obtain a plurality of waveforms from the PPG signal received for the pre-specified time. FIG. 6B illustrates a situation in which four waveforms P1, P2, P3, and P4 are obtained (640). In addition, the processor 250 may compare the obtained four waveforms with the selected waveform template (650), and may measure similarity of each waveform (660). In FIG. 6B, P1s, P2s, P3s, and P4s refer to similarities measured regarding the waveform P1, waveform P2, waveform P3, and waveform P4, respectively. The similarity "1.0" of the waveform P4 of FIG. 6B may be interpreted as meaning that the PPG signal and the waveform template are almost the same, and the similarity decreases in order of the waveform P1, waveform P3, and waveform P2. The similarity may be measured by comparing the feature point of the waveform template and the feature point of the obtained waveform. For example, the feature point used to measure the similarity may include at least one of the feature points described above through FIG. 4C.

According to the invention, the electronic device (for example, the processor 250) determines whether the similarity of the PPG signal falls within a pre-specified range in operation 614. The processor 250 determines whether the similarity of the PPG signal falls within the pre-specified range, based on the number of waveforms having similarities greater than or equal to a threshold value.

Referring to FIG. 6B, when the number of waveforms having similarities greater than or equal to a threshold value (for example, 0.7) is greater than or equal to a reference number of waveforms (for example, 3), the processor 250 determines that the similarity of the PPG signal falls within the pre-specified range. According to another embodiment of the disclosure, the processor 250 may determine whether the similarity of the PPG signal falls within the pre-specified range, based on a similarity mean value of the waveforms obtained from the PPG signal. Referring to FIG. 6B, when a mean value of the similarities P1s, P2s, P3s, and P4s of the waveform P1, waveform P2, waveform P3, and waveform P4 is greater than or equal to a reference mean value (for example, 0.7), the processor 250 may determine that the similarity of the PPG signal falls within the pre-specified range.

According to the invention, when the similarity between the selected waveform template and the extracted comparison waveform falls within the pre-specified range, the electronic device (for example, the processor 250) determines a blood pressure value based on the waveform template in operation 616. For example, the processor 250 may determine a previously measured blood pressure value (for example, at least one of a systolic blood pressure value, a diastolic blood pressure value, a mean arterial pressure value) included in the waveform template, as a current blood pressure value of the user. According to various embodiments of the disclosure, since the blood pressure value is determined based on the waveform template, the operation of generating a representative waveform by using the PPG signal can be omitted. Accordingly, time required to measure blood pressure by the electronic device can be reduced. In another example, the processor 250 may calculate a current blood pressure value of the user by analyzing the feature point of the selected waveform template, namely, of the previously generated waveform template.

According to the invention, when the similarity between the selected waveform template and the extracted comparison waveform falls out of the pre-specified range, the electronic device (for example, the processor 250) performs the operation of obtaining a representative waveform by processing the PPG signal in operation 618. In addition, the processor 250 generates the representative waveform by processing the PPG signal, and then, measures the blood pressure value of the user by using the generated representative waveform, and stores an additional waveform template based on the generated representative waveform, as in operations 620 and 622.

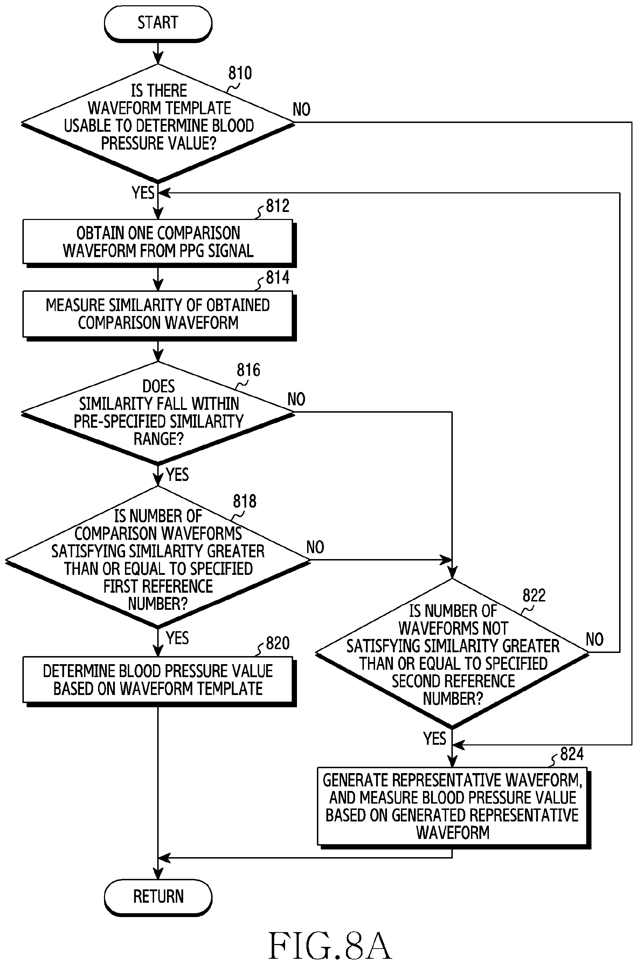

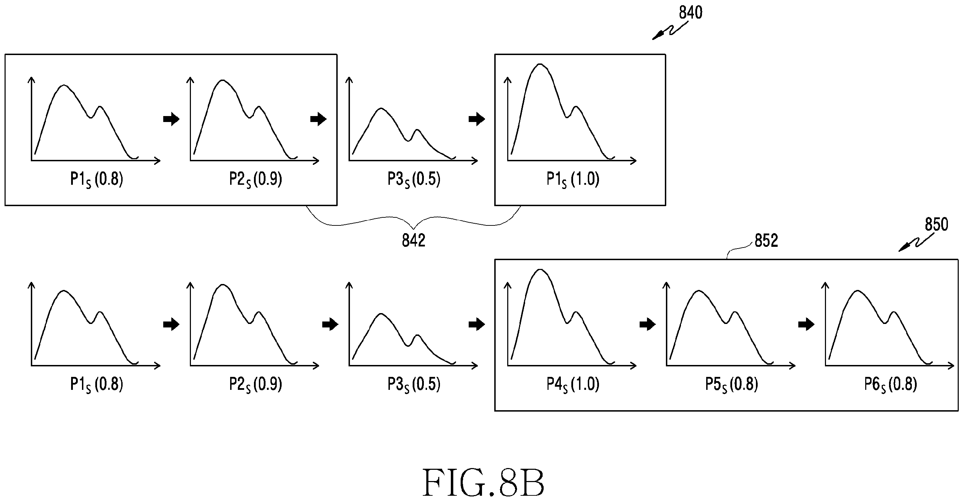

FIG. 8A is a flowchart for determining a blood pressure value based on a waveform template in an electronic device according to an embodiment of the disclosure. FIG. 8B is a view illustrating an operation of measuring similarity of a comparison waveform according to an embodiment of the disclosure. Operations of FIG. 8A, which will be described below, indicate various embodiments of operations 520 and 530 of FIG. 5. In the following embodiments of the disclosure, respective operations may be performed in sequence, but may not necessarily be performed in sequence. For example, the order of the operations may be changed, or at least two operations may be performed in parallel. The electronic device of FIGS. 8A and 8B may be the electronic device 200 of FIG. 2.

Referring to FIG. 8A, the electronic device (for example, the processor 250 of FIG. 2) may determine whether there exists a waveform template to be used to determine a blood pressure value in operation 810. It may be determined whether there exists a waveform template in the same or similar method as or to the method in operation 610 of FIG. 6 described above.

According to various embodiments of the disclosure, when it is determined that there does not exist a waveform template to be used to determine a blood pressure value, the electronic device (for example, the processor 250) may generate a representative waveform by processing a PPG signal in operation 822, and may measure a blood pressure value of the user by using the generated representative waveform. For example, the processor 250 may perform an operation associated with at least one of operations 618 to 622 of FIG. 6 described above.

According to various embodiments of the disclosure, when it is determined that there exists a waveform template to be used to determine a blood pressure value, the electronic device (for example, the processor 250) may extract one comparison waveform from the PPG signal in operation 812. According to an embodiment of the disclosure, the comparison waveform may be a waveform that may be used to measure similarity with the waveform template.

According to various embodiments of the disclosure, the electronic device (for example, the processor 250) may measure similarity of the obtained comparison waveform in operation 814. The operation of measuring similarity may be performed every time the comparison waveform is extracted from the PPG signal. For example, the processor 250 may measure the similarity by comparing a feature point of the extracted comparison waveform and a feature point of the selected waveform template. For example, the similarity may be determined in the same or similar method as or to the method in operation 612 of FIG. 6.

According to various embodiments of the disclosure, the electronic device (for example, the processor 250) may determine whether the similarity of the PPG waveform falls within a pre-specified similarity range in operation 816.

According to various embodiments of the disclosure, when the similarity of the PPG waveform falls within the pre-specified similarity range, the electronic device (for example, the processor 250) may determine whether the number of comparison waveforms satisfying the similarity is greater than or qual to a pre-specified first reference number in operation 818. The first reference number may be a reference value which is used to determine that the waveform of the PPG signal and the waveform template are the same or similar as or to each other. In addition, the first reference number may be interpreted as a value for determining that blood pressure has been measured in the past in a similar condition to a current blood pressure measuring condition. According to an embodiment of the disclosure, the first reference number may be associated with the number of accumulated comparison waveforms satisfying the similarity. For example, when it is assumed that the pre-specified similarity range is 0.7-1.0, and the pre-specified first reference number is 3, the processor 250 may repeat the operation of extracting the comparison waveform until the number of comparison waveforms satisfying similarity is greater than or equal to the third reference number (840), as shown in FIG. 8B. When three comparison waveforms in total (first waveform, second waveform, and fourth waveform) 842 satisfying the similarity are extracted as shown in the drawing, the processor 250 may determine that the number of comparison waveforms satisfying the similarity satisfies (or exceeds) the pre-specified first reference number. Similarly, to FIG. 6B, P1s, P2s, P3s, and P4s of FIG. 8B refer to similarities measured regarding the waveform P1, waveform P2, waveform P3, and waveform P4, respectively. According to another embodiment of the disclosure, the first reference number may be associated with the number of continuous comparison waveforms satisfying the similarity. For example, when it is assumed that the pre-specified similarity range is 0.7-1.0 and the pre-specified first reference number is 3, the processor 250 may repeat the operation of extracting the comparison waveform until the number of continuous comparison waveforms satisfying the similarity is greater than or equal to the first reference number (850) as shown in FIG. 8B. When three comparison waveforms in total satisfying the similarity (fourth waveform, fifth waveform, and sixth waveform) 852 are continuously extracted as shown in the drawing, the processor 250 may determine that the number of comparison waveforms satisfying the similarity satisfies (or exceeds) the pre-specified first reference number. The method of extracting continuous comparison waveforms satisfying similarity (850) may measure similarity of the PPG waveform having reliability, in comparison to the method of extracting accumulated comparison waveforms satisfying similarity (840). On the other hand, the method of extracting accumulated comparison waveforms satisfying similarity (840) provides the advantage of measuring similarity of the PPG waveform rapidly, in comparison to the method of extracting continuous comparison waveforms satisfying similarity (850).

According to various embodiments of the disclosure, when the number of comparison waveforms satisfying the similarity is greater than or equal to the pre-specified first reference number, the electronic device (for example, the processor 250) may determine a blood pressure value based on the waveform template in operation 820. For example, the processor 250 may determine a previously measured blood pressure value included in the waveform template as a current blood pressure value of the user.

According to various embodiment of the disclosure, when the similarity between the selected waveform template and the extracted comparison waveform falls out of the pre-specified similarity range, or the number of comparison waveforms satisfying the similarity is less than the pre-specified first reference number, the electronic device (for example, the processor 250) may determine whether the number of waveforms not satisfying the similarity exceeds a specified second reference number in operation 822. The second reference number is a reference value which is used to determine that the PPG waveform and the waveform template are not the same as or similar to each other. In addition, the second reference number may be interpreted as a value for determining that blood pressure has not been measured in the past in a condition similar to the current blood pressure measuring condition.

According to various embodiments of the disclosure, when the number of waveforms not satisfying the similarity does not exceed the specified second reference number, the electronic device (for example, the processor 250) may repeat the operation of extracting the comparison waveform and comparing the similarity. According to an embodiment of the disclosure, the processor 250 may determine that there is a possibility that blood pressure has been measured in the past in the condition similar to the current blood pressure measuring condition, and may repeat an operation associated with at least one of operations 812 to 818.

According to various embodiments of the disclosure, when the number of waveforms not satisfying the similarity exceeds the specified second reference number, the electronic device (for example, the processor 250) may generate a representative waveform by processing the PPG signal, and may measure a blood pressure value of the user by analyzing the generated representative waveform in operation 824. For example, the processor 250 may determine that blood pressure has not been measured in the past in the condition similar to the current blood pressure measuring condition, and may generate a new representative waveform and may measure a blood pressure value of the user.

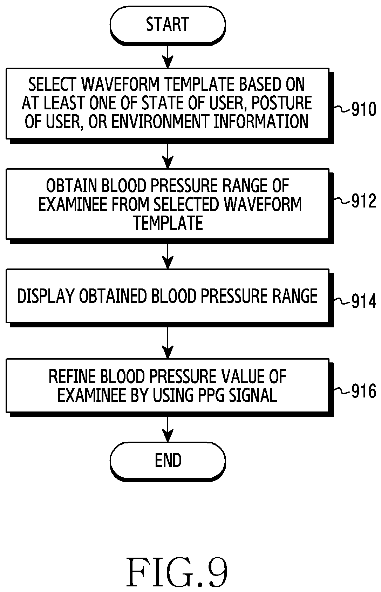

FIG. 9 is a flowchart for determining a blood pressure value in an electronic device according to an embodiment of the disclosure.

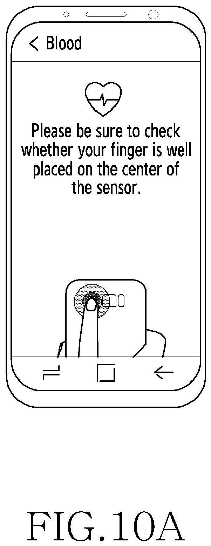

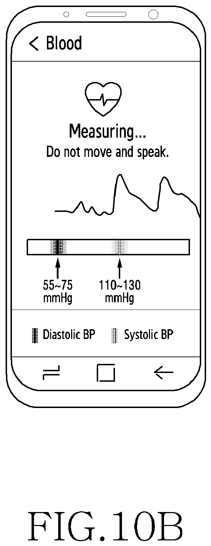

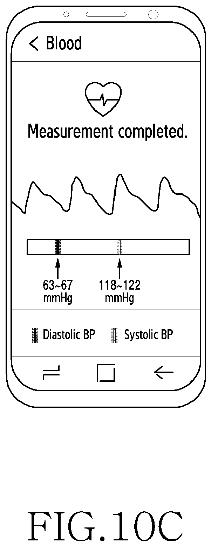

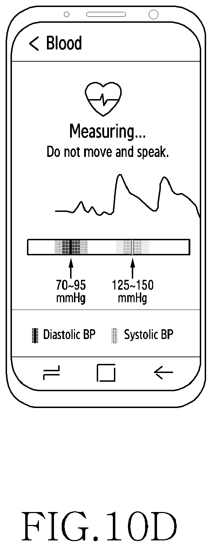

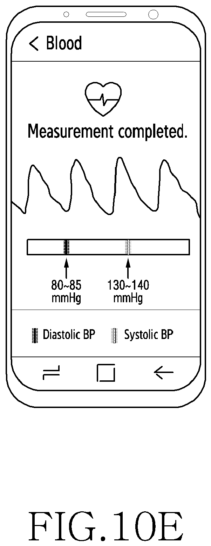

FIG. 10A is a view illustrating a screen of an electronic device performing an operation of determining a blood pressure value according to an embodiment of the disclosure, FIG. 10B is a view illustrating a screen of an electronic device performing an operation of determining the blood pressure value according to an embodiment of the disclosure, FIG. 10C is a view illustrating a screen of an electronic device performing an operation of determining a blood pressure value according to an embodiment of the disclosure, FIG. 10D is a view illustrating a screen of an electronic device performing an operation of determining a blood pressure value according to an embodiment of the disclosure, and FIG. 10E is a view illustrating a screen of an electronic device performing an operation of determining a blood pressure value according to an embodiment of the disclosure. In the following embodiments of the disclosure, respective operations may be performed in sequence, but may not necessarily be performed in sequence. For example, the order of the operations may be changed, or at least two operations may be performed in parallel. The electronic device of FIG. 9 may be the electronic device 200 of FIG. 2.

Referring to FIG. 9, in operation 910, the electronic device (for example, the processor 250 of FIG. 2) according to various embodiments may select a waveform template by using data associated with at least one of a body (psychological) state of an examinee (for example, the user of the electronic device), a posture of the examinee, or environment information (for example, location information, temperature/humidity information, and the like). The waveform template may be selected based on a user's heart rate or a posture of the electronic device, and the processor 250 may perform the same or similar operations as or to operations 510 and 520 of FIG. 5 described above to select the waveform template. In addition, the waveform template may be selected in response to a part of the body of the user (for example, a finger) contacting or approaching a biometric sensor (for example, the first sensor module 210) being detected as shown in FIG. 10A.

According to various embodiments of the disclosure, the electronic device (for example, the processor 250) may obtain a blood pressure range of the examinee from the selected waveform template in operation 912. According to an embodiment of the disclosure, the processor 250 may obtain a PPG signal from the biometric sensor (for example, the first sensor module 210 of FIG. 2), and, when similarity between the waveform of the obtained PPG signal and a waveform of the selected waveform template falls within a pre-specified range, the processor 250 may obtain a blood pressure range from the selected waveform template, as in operation 530 of FIG. 5 described above.

According to various embodiments of the disclosure, the electronic device (for example, the processor 250) may display the obtained blood pressure range in operation 914. For example, a blood pressure value obtained from the selected waveform template is a blood pressure value which was measured in the past, and the processor 250 may predict the range of blood pressure values to be determined by using the past blood pressure value, and may display the range by using a number, a graph, or the like. When a waveform template related to an examinee without a motion is selected, the processor 250 may display a range of systolic blood pressure (for example, 110 mmHg-130 mmHg), and a range of diastolic blood pressure (for example, 55 mmHg-75 mmHg) as shown in FIG. 10B. In addition, when a waveform template related to an examinee with a motion is selected, the processor 250 may display a range of systolic blood pressure (for example, 125 mmHg-150 mmHg), and a range of diastolic blood pressure (for example, 70 mmHg-95 mmHg) as shown in FIG. 10D.

According to various embodiments of the disclosure, the electronic device (for example, the processor 250) may refine the blood pressure value of the examinee by using the PPG signal in operation 916. For example, the processor 250 may generate a representative waveform by processing the PPG signal, and may refine (or display) the blood pressure value of the user by analyzing the generated representative waveform as in operations 618 and 620. The refined blood pressure value is a blood pressure value which is really measured based on the PPG signal, and may have higher accuracy than that of the blood pressure value from the waveform template. For example, when a PPG signal is obtained from an examinee without a motion, the processor 250 may display a systolic blood pressure value (for example, 118 mmHg-122 mmHg), and a diastolic blood pressure value (for example, 63 mmHg-67 mmHg), which are measured by using the PPG signal, as shown FIG. 10C. In another example, when a PPG signal is obtained from an examinee with a motion, the processor 250 may display a systolic blood pressure value (for example, 130 mmHg-140 mmHg), and a diastolic blood pressure value (for example, 80 mmHg-85 mmHg) which are measured by using the PPG signal, as shown in FIG. 10E. The processor 250 may maintain the display of the blood pressure range until the blood pressure value is refined, and may refine the blood pressure value by using a number, a graph, a table, or the like.

According to various embodiments of the disclosure, the electronic device (for example, the processor 250) may add a waveform template by using the PPG signal which is used to refine the blood pressure value of the examinee. The processor 250 may add a waveform template including at least one of the waveform of the PPG signal, the refined blood pressure value, the state of the user, the posture of the user, or the measuring environment every time the blood pressure value is refined, and accordingly, the accuracy of determining a blood pressure value by using the waveform template can be enhanced.

According to various embodiments of the disclosure, an operating method of an electronic device (for example, the electronic device 200 of FIG. 2) may include, obtaining information related to at least one of a state of a user, a posture of the user, or a measuring environment, selecting one waveform template from a plurality of waveform templates stored in the electronic device, based on the obtained information, and determining a blood pressure value of the user by using the selected waveform template and a measured biometric signal. According to an embodiment of the disclosure, the biometric signal may include a PPG signal. According to an embodiment of the disclosure, the information related to the state of the user may include a heart rate. According to an embodiment of the disclosure, the information related to the posture of the user may include at least one of a state in which blood pressure is measured by using the left or right hand, a standing state, a sitting state, a dynamic state, a static state, a sleeping state, a resting state, an exercising state, or a state after exercising. According to an embodiment of the disclosure, the information related to the measuring environment may include at least one of a location of the electronic device, a temperature or humidity when blood pressure is measured.

According to various embodiments of the disclosure, the operating method of the electronic device (for example, the electronic device 200 of FIG. 2) may include determining whether the selected waveform template is used, based on a similarity indicating how much the selected waveform template and the biometric signal are similar to each other.

According to various embodiments of the disclosure, the operating method of the electronic device (for example, the electronic device 200 of FIG. 2) may include, when the similarity indicating how much the selected waveform template and the biometric signal are similar to each other falls within a specified similarity range, determining the blood pressure value of the user based on the selected waveform template.

According to various embodiments of the disclosure, the operating method of the electronic device (for example, the electronic device 200 of FIG. 2) may include determining the blood pressure value of the user based on the selected waveform template, and then adding a waveform template including the biometric signal.

According to various embodiments of the disclosure, the operating method of the electronic device (for example, the electronic device 200 of FIG. 2) may include, when the similarity indicating how much the selected waveform template and the biometric signal are similar to each other does not fall within the specified similarity range, generating a representative waveform by using the biometric signal, measuring the blood pressure value of the user based on the generated representative waveform, and adding a waveform template corresponding to the generated representative waveform.

According to various embodiments of the disclosure, adding the waveform template may include generating the representative waveform based on at least one of the state of the user, the posture of the user, or the measuring environment, and adding the generated representative waveform to the waveform template.

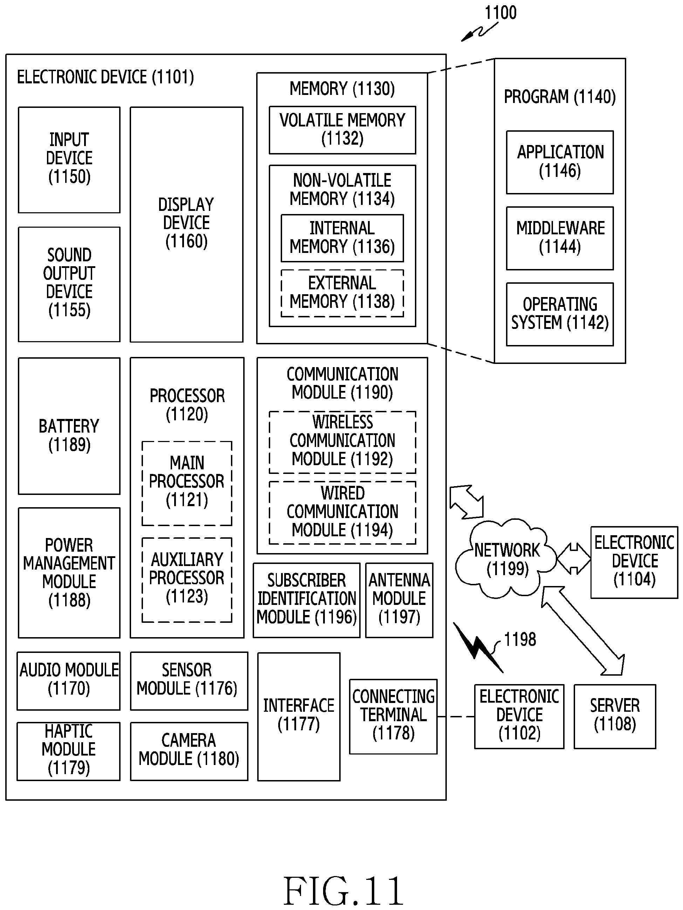

FIG. 11 is a block diagram of an electronic device in a network environment according to an embodiment of the disclosure. The electronic device of FIG. 11 may be the electronic device 200 of FIG. 2.

Referring to FIG. 11, an electronic device 1101 in a network environment 1100 may communicate with an electronic device 1102 via a first network 1198 (e.g., a short-range wireless communication network), or an electronic device 1104 or a server 1108 via a second network 1199 (e.g., a long-range wireless communication network). According to an embodiment of the disclosure, the electronic device 1101 may communicate with the electronic device 1104 via the server 1108. According to an embodiment of the disclosure, the electronic device 1101 may include a processor 1120 (e.g., the processor 120 in FIG. 1A), memory 1130 (e.g., the memory 130 in FIG. 1A), an input device 1150, a sound output device 1155, a display device 1160 (e.g., the display 160 in FIG. 1A), an audio module 1170, a sensor module 1176, an interface 1177, a haptic module 1179, a camera module 1180, a power management module 1188, a battery 1189, a communication module 1190 (e.g., the communication interface 170 in FIG. 1A), a subscriber identification module (SIM) 1196, or an antenna module 1197. In some embodiments of the disclosure, at least one (e.g., the display device 1160 or the camera module 1180) of the components may be omitted from the electronic device 1101, or one or more other components may be added in the electronic device 1101. In some embodiments of the disclosure, some of the components may be implemented as single integrated circuitry. For example, the sensor module 1176 (e.g., a fingerprint sensor, an iris sensor, or an illuminance sensor) may be implemented as embedded in the display device 1160 (e.g., a display).

The processor 1120 may execute, for example, software (e.g., a program 1140) to control at least one other component (e.g., a hardware or software component) of the electronic device 1101 coupled with the processor 1120, and may perform various data processing or computation. According to one embodiment of the disclosure, as at least part of the data processing or computation, the processor 1120 may load a command or data received from another component (e.g., the sensor module 1176 or the communication module 1190) in volatile memory 1132, process the command or the data stored in the volatile memory 1132, and store resulting data in non-volatile memory 1134. According to an embodiment of the disclosure, the processor 1120 may include a main processor 1121 (e.g., a central processing unit (CPU) or an application processor (AP)), and an auxiliary processor 1123 (e.g., a graphics processing unit (GPU), an image signal processor (ISP), a sensor hub processor, or a communication processor (CP)) that is operable independently from, or in conjunction with, the main processor 1121. Additionally or alternatively, the auxiliary processor 1123 may be adapted to consume less power than the main processor 1121, or to be specific to a specified function. The auxiliary processor 1123 may be implemented as separate from, or as part of the main processor 1121.

The auxiliary processor 1123 may control at least some of functions or states related to at least one component (e.g., the display device 1160, the sensor module 1176, or the communication module 1190) among the components of the electronic device 1101, instead of the main processor 1121 while the main processor 1121 is in an inactive (e.g., sleep) state, or together with the main processor 1121 while the main processor 1121 is in an active state (e.g., executing an application). According to an embodiment of the disclosure, the auxiliary processor 1123 (e.g., an ISP or a CP) may be implemented as part of another component (e.g., the camera module 1180 or the communication module 1190) functionally related to the auxiliary processor 1123.

The memory 1130 may store various data used by at least one component (e.g., the processor 1120 or the sensor module 1176) of the electronic device 1101. The various data may include, for example, software (e.g., the program 1140) and input data or output data for a command related thererto. The memory 1130 may include the volatile memory 1132 or the non-volatile memory 1134.

The program 1140 may be stored in the memory 1130 as software, and may include, for example, an operating system (OS) 1142, middleware 1144, or an application 1146.

The input device 1150 may receive a command or data to be used by other component (e.g., the processor 1120) of the electronic device 1101, from the outside (e.g., a user) of the electronic device 1101. The input device 1150 may include, for example, a microphone, a mouse, or a keyboard.

The sound output device 1155 may output sound signals to the outside of the electronic device 1101. The sound output device 1155 may include, for example, a speaker or a receiver. The speaker may be used for general purposes, such as playing multimedia or playing record, and the receiver may be used for an incoming calls. According to an embodiment of the disclosure, the receiver may be implemented as separate from, or as part of the speaker.

The display device 1160 may visually provide information to the outside (e.g., a user) of the electronic device 1101. The display device 1160 may include, for example, a display, a hologram device, or a projector and control circuitry to control a corresponding one of the display, hologram device, and projector. According to an embodiment of the disclosure, the display device 1160 may include touch circuitry adapted to detect a touch, or sensor circuitry (e.g., a pressure sensor) adapted to measure the intensity of force incurred by the touch.

The audio module 1170 may convert a sound into an electrical signal and vice versa. According to an embodiment of the disclosure, the audio module 1170 may obtain the sound via the input device 1150, or output the sound via the sound output device 1155 or a headphone of an external electronic device (e.g., an electronic device 1102) directly (e.g., wired connection) or wirelessly coupled with the electronic device 1101.

The sensor module 1176 may detect an operational state (e.g., power or temperature) of the electronic device 1101 or an environmental state (e.g., a state of a user) external to the electronic device 1101, and then generate an electrical signal or data value corresponding to the detected state. According to an embodiment of the disclosure, the sensor module 1176 may include, for example, a gesture sensor, a gyro sensor, an atmospheric pressure sensor, a magnetic sensor, an acceleration sensor, a grip sensor, a proximity sensor, a color sensor, an IR sensor, a biometric sensor, a temperature sensor, a humidity sensor, or an illuminance sensor.

The interface 1177 may support one or more specified protocols to be used for the electronic device 1101 to be coupled with the external electronic device (e.g., the electronic device 1102) directly (e.g., wired connection) or wirelessly. According to an embodiment of the disclosure, the interface 1177 may include, for example, a high definition multimedia interface (HDMI), a universal serial bus (USB) interface, a secure digital (SD) card interface, or an audio interface.

A connecting terminal 1178 may include a connector via which the electronic device 1101 may be physically connected with the external electronic device (e.g., the electronic device 1102). According to an embodiment of the disclosure, the connecting terminal 1178 may include, for example, a HDMI connector, a USB connector, a SD card connector, or an audio connector (e.g., a headphone connector).

The haptic module 1179 may convert an electrical signal into a mechanical stimulus (e.g., a vibration or a movement) or electrical stimulus which may be recognized by a user via his tactile sensation or kinesthetic sensation. According to an embodiment of the disclosure, the haptic module 1179 may include, for example, a motor, a piezoelectric element, or an electric stimulator.

The camera module 1180 may capture a still image or moving images. According to an embodiment of the disclosure, the camera module 1180 may include one or more lenses, image sensors, ISPs, or flashes.

The power management module 1188 may manage power supplied to the electronic device 1101. According to one embodiment of the disclosure, the power management module 1188 may be implemented as at least part of, for example, a power management integrated circuit (PMIC).

The battery 1189 may supply power to at least one component of the electronic device 1101. According to an embodiment of the disclosure, the battery 1189 may include, for example, a primary cell which is not rechargeable, a secondary cell which is rechargeable, or a fuel cell.

The communication module 1190 may support establishing a direct (e.g., wired) communication channel or a wireless communication channel between the electronic device 1101 and the external electronic device (e.g., the electronic device 1102, the electronic device 1104, or the server 1108) and performing communication via the established communication channel. The communication module 1190 may include one or more CPs that are operable independently from the processor 1120 (e.g., the AP) and supports a direct (e.g., wired) communication or a wireless communication. According to an embodiment of the disclosure, the communication module 1190 may include a wireless communication module 1192 (e.g., a cellular communication module, a short-range wireless communication module, or a global navigation satellite system (GNSS) communication module) or a wired communication module 1194 (e.g., a local area network (LAN) communication module or a power line communication (PLC) module). A corresponding one of these communication modules may communicate with the external electronic device via the first network 1198 (e.g., a short-range communication network, such as Bluetooth^{™}, wireless-fidelity (Wi-Fi) direct, or infrared data association (IrDA)) or the second network 1199 (e.g., a long-range communication network, such as a cellular network, the Internet, or a computer network (e.g., LAN or wide area network (WAN)). These various types of communication modules may be implemented as a single component (e.g., a single chip), or may be implemented as multi components (e.g., multi chips) separate from each other.

The wireless communication module 1192 may identify and authenticate the electronic device 1101 in a communication network, such as the first network 1198 or the second network 1199, using subscriber information (e.g., international mobile subscriber identity (IMSI)) stored in the SIM 1196.

The antenna module 1197 may transmit or receive a signal or power to or from the outside (e.g., the external electronic device) of the electronic device 1101. According to an embodiment of the disclosure, the antenna module 1197 may include one or more antennas, and, therefrom, at least one antenna appropriate for a communication scheme used in the communication network, such as the first network 1198 or the second network 1199, may be selected, for example, by the communication module 1190 (e.g., the wireless communication module 1192). The signal or the power may then be transmitted or received between the communication module 1190 and the external electronic device via the selected at least one antenna.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment of the disclosure, commands or data may be transmitted or received between the electronic device 1101 and the electronic device 1104 via the server 1108 coupled with the second network 1199. Each of the electronic devices 1102 and 1104 may be a device of a same type as, or a different type, from the electronic device 1101. According to an embodiment of the disclosure, all or some of operations to be executed at the electronic device 1101 may be executed at one or more of the external electronic devices 1102, 1104, or 1108. For example, if the electronic device 1101 should perform a function or a service automatically, or in response to a request from a user or another device, the electronic device 1101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 1101. The electronic device 1101 may provide the outcome, with or without further processing of the outcome, as at least part of a reply to the request. To that end, a cloud computing, distributed computing, or client-server computing technology may be used, for example.

The electronic device according to various embodiments may be one of various types of electronic devices. The electronic devices may include, for example, a portable communication device (e.g., a smart phone), a computer device, a portable multimedia device, a portable medical device, a camera, a wearable device, or a home appliance. According to an embodiment of the disclosure, the electronic devices are not limited to those described above.

It should be appreciated that various embodiments of the disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wired connection), wirelessly, or via a third element.

As used herein, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment of the disclosure, the module may be implemented in a form of an application-specific integrated circuit (ASIC).

Various embodiments as set forth herein may be implemented as software (e.g., the program 1140) including one or more instructions that are stored in a storage medium (e.g., internal memory 1136 or external memory 1138) that is readable by a machine (e.g., the electronic device 1101). For example, a processor (e.g., the processor 1120) of the machine (e.g., the electronic device 1101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment of the disclosure, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., Play Store^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments of the disclosure, each component (e.g., a module or a program) of the above-described components may include a single entity or multiple entities. According to various embodiments of the disclosure, one or more of the above-described components may be omitted, or one or more other components may be added. Alternatively or additionally, a plurality of components (e.g., modules or programs) may be integrated into a single component. In such a case, according to various embodiments of the disclosure, the integrated component may still perform one or more functions of each of the plurality of components in the same or similar manner as they are performed by a corresponding one of the plurality of components before the integration. According to various embodiments of the disclosure, operations performed by the module, the program, or another component may be carried out sequentially, in parallel, repeatedly, or heuristically, or one or more of the operations may be executed in a different order or omitted, or one or more other operations may be added.

According to various embodiments of the disclosure, the operation of the electronic device and the apparatus may select one waveform template from the plurality of waveform templates, based on at least one of a user's state, a user's posture, or a measuring environment, and may determine a blood pressure value of the user by using the selected waveform template, such that time required to measure blood pressure can be reduced.

## Claims

1. An electronic device (200) comprising:
at least one sensor (210; 220) including a biometric sensor (210);
a processor (250); and
a memory (230) operatively connected with the processor (250),
wherein, when being executed, the memory (230) stores instructions that cause the processor (250) to:
obtain information from the at least one sensor (210; 220) related to at least one of a state of a user, a posture of the user, or a measuring environment, in response to a part of the user's body contacting or approaching the biometric sensor (210) being detected,
select one waveform template from a plurality of waveform templates stored in the memory, based on at least one of the state of the user, the posture of the user, or the measuring environment,
measure a similarity by comparing a photoplethysmogram (PPG) signal obtained from the at least one sensor (210; 220) and the selected waveform template,
determine whether the similarity of the PPG signal falls within a pre-specified range, based on whether the number of waveforms having similarities greater than or equal to a threshold value is greater than or equal to a reference number of waveforms,
if the similarity of the PPG signal falls within the pre-specified range:
determine a blood pressure value of the user by using the selected waveform template,
if the similarity of the PPG signal does not fall within the pre-specified range:
generate a representative waveform by using the PPG signal,
measure the blood pressure value of the user based on the generated representative waveform, and
add a waveform template corresponding to the generated representative waveform in the memory.

2. The electronic device (200) of claim 1, wherein the at least one sensor (210; 220) comprises:
the biometric sensor (210) configured to obtain the information related to the state of the user; and
an additional sensor (220) configured to obtain the information related to at least one of the posture of the user or the measuring environment,
wherein the additional sensor (220) comprises a gesture sensor, a gyro sensor (222), an atmospheric pressure sensor, a magnetic sensor, an acceleration sensor (221), a grip sensor, a proximity sensor (223), a color sensor, an infrared (IR) sensor, a temperature sensor, a humidity sensor (225), or an illuminance sensor.

3. The electronic device (200) of claim 1, wherein the information related to the state of the user comprises a heart rate.

4. The electronic device (200) of claim 1, wherein the information related to the measuring environment comprises at least one of a location of the electronic device, a temperature, or humidity when blood pressure is measured.

5. The electronic device (200) of claim 1, wherein the instructions comprise an instruction that causes the processor (250) to:
generate the representative waveform based on at least one of the state of the user, the posture of the user, or the measuring environment when adding the waveform template corresponding to the representative waveform, and
add the generated representative waveform to the waveform template.

6. An operating method of an electronic device, the method comprising:
obtaining information from at least one sensor including a biometric sensor related to at least one of a state of a user, a posture of the user, or a measuring environment, in response to a part of the user's body contacting or approaching the biometric sensor being detected;
selecting one waveform template from a plurality of waveform templates stored in the electronic device, based on the obtained information;
measuring a similarity by comparing a photoplethysmogram (PPG) signal obtained from the at least one sensor and the selected waveform template;
determining whether the similarity of the PPG signal falls within a pre-specified range, based on whether the number of waveforms having similarities greater than or equal to a threshold value is greater than or equal to a reference number of waveforms;
if the similarity of the PPG signal falls within the pre-specified range:
determining a blood pressure value of the user by using the selected waveform template and a measured biometric signal;
if the similarity of the PPG signal does not fall within the pre-specified range:
generating a representative waveform by using the PPG signal,
measuring the blood pressure value of the user based on the generated representative waveform, and
adding a waveform template corresponding to the generated representative waveform in the memory.

7. The method of claim 6, wherein the information related to the state of the user comprises a heart rate.

8. The method of claim 6, wherein the information related to the measuring environment comprises at least one of a location of the electronic device, a temperature, or humidity when blood pressure is measured.

9. The method of claim 6, wherein the information related to the posture of the user comprises at least one of a state in which blood pressure is measured by using the left or right hand, a standing state, a sitting state, a dynamic state, a static state, a sleeping state, a resting state, an exercising state, or a state after exercising.

## Patentansprüche

1. Elektronische Vorrichtung (200), die Folgendes umfasst:
mindestens einen Sensor (210; 220), der einen biometrischen Sensor (210) enthält;
einen Prozessor (250); und
einen Speicher (230), der mit dem Prozessor (250) operativ verbunden ist,
wobei der Speicher (230), wenn er ausgeführt wird, Anweisungen speichert, die den Prozessor (250) zu Folgendem veranlassen:
Erhalten von Informationen von dem mindestens einen Sensor (210; 220) in Bezug auf einen Zustand eines Benutzers, eine Haltung des Benutzers und/oder eine Messumgebung als Reaktion darauf, dass ein Teil des Körpers des Benutzers erkannt wird, der den biometrischen Sensor (210) berührt oder sich diesem nähert,
Auswählen einer Wellenformvorlage aus einer Vielzahl von Wellenformvorlagen, die in dem Speicher gespeichert sind, basierend auf dem Zustand des Benutzers, der Haltung des Benutzers und/oder der Messumgebung,
Messen einer Ähnlichkeit durch Vergleichen eines Photoplethysmogramm(PPG)-Signals, das von dem mindestens einen Sensor (210; 220) erhalten wird, und der ausgewählten Wellenformvorlage,
Bestimmen, ob die Ähnlichkeit des PPG-Signals innerhalb eines vorgegebenen Bereichs liegt, basierend darauf, ob die Anzahl von Wellenformen mit Ähnlichkeiten, die größer als oder gleich einem Schwellenwert sind, größer als oder gleich einer Referenzanzahl von Wellenformen ist,
wenn die Ähnlichkeit des PPG-Signals innerhalb des vorgegebenen Bereichs liegt:
Bestimmen eines Blutdruckwerts des Benutzers unter Verwendung der ausgewählten Wellenformvorlage,
wenn die Ähnlichkeit des PPG-Signals nicht innerhalb des vorgegebenen Bereichs liegt:
Erzeugen einer repräsentativen Wellenform unter Verwendung des PPG-Signals,
Messen des Blutdruckwerts des Benutzers basierend auf der erzeugten repräsentativen Wellenform, und
Hinzufügen einer Wellenformvorlage, die der erzeugten repräsentativen Wellenform in dem Speicher entspricht.

2. Elektronische Vorrichtung (200) nach Anspruch 1, wobei der mindestens eine Sensor (210; 220) Folgendes umfasst:
den biometrischen Sensor (210), der so konfiguriert ist, dass er die Informationen in Bezug auf den Zustand des Benutzers erhält; und
einen zusätzlichen Sensor (220), der so konfiguriert ist, dass er die Informationen in Bezug auf die Haltung des Benutzers und/oder die Messumgebung erhält,
wobei der zusätzliche Sensor (220) einen Gestensensor, einen Gyrosensor (222), einen Atmosphärendrucksensor, einen Magnetsensor, einen Beschleunigungssensor (221), einen Griffsensor, einen Näherungssensor (223), einen Farbsensor, einen Infrarot(IR)-Sensor, einen Temperatursensor, einen Luftfeuchtigkeitssensor (225) oder einen Beleuchtungsstärkesensor umfasst.

3. Elektronische Vorrichtung (200) nach Anspruch 1, wobei die Informationen in Bezug auf den Zustand des Benutzers eine Herzfrequenz umfassen.

4. Elektronische Vorrichtung (200) nach Anspruch 1, wobei die Informationen in Bezug auf die Messumgebung einen Standort der elektronischen Vorrichtung, eine Temperatur und/oder eine Luftfeuchtigkeit bei der Blutdruckmessung umfassen.

5. Elektronische Vorrichtung (200) nach Anspruch 1, wobei die Anweisungen eine Anweisung umfassen, die den Prozessor (250) zu Folgendem veranlasst:
Erzeugen der repräsentativen Wellenform basierend auf dem Zustand des Benutzers, der Haltung des Benutzers und/oder der Messumgebung beim Hinzufügen der Wellenformvorlage, die der repräsentativen Wellenform entspricht, und
Hinzufügen der erzeugten repräsentativen Wellenform zu der Wellenformvorlage.

6. Betriebsverfahren einer elektronischen Vorrichtung, wobei das Verfahren Folgendes umfasst:
Erhalten von Informationen von mindestens einem Sensor, der einen biometrischen Sensor enthält, in Bezug auf einen Zustand eines Benutzers, eine Haltung des Benutzers und/oder eine Messumgebung als Reaktion darauf, dass ein Teil des Körpers des Benutzers erkannt wird, der den biometrischen Sensor berührt oder sich diesem nähert;
Auswählen einer Wellenformvorlage aus einer Vielzahl von Wellenformvorlagen, die in der elektronischen Vorrichtung gespeichert sind, basierend auf den erhaltenen Informationen;
Messen einer Ähnlichkeit durch Vergleichen eines Photoplethysmogramm(PPG)-Signals, das von dem mindestens einen Sensor erhalten wird, und der ausgewählten Wellenformvorlage;
Bestimmen, ob die Ähnlichkeit des PPG-Signals innerhalb eines vorgegebenen Bereichs liegt, basierend darauf, ob die Anzahl von Wellenformen mit Ähnlichkeiten, die größer als oder gleich einem Schwellenwert sind, größer als oder gleich einer Referenzanzahl von Wellenformen ist;
wenn die Ähnlichkeit des PPG-Signals innerhalb des vorgegebenen Bereichs liegt:
Bestimmen eines Blutdruckwerts des Benutzers unter Verwendung der ausgewählten Wellenformvorlage und eines gemessenen biometrischen Signals;
wenn die Ähnlichkeit des PPG-Signals nicht innerhalb des vorgegebenen Bereichs liegt:
Erzeugen einer repräsentativen Wellenform unter Verwendung des PPG-Signals,
Messen des Blutdruckwerts des Benutzers basierend auf der erzeugten repräsentativen Wellenform, und
Hinzufügen einer Wellenformvorlage, die der erzeugten repräsentativen Wellenform in dem Speicher entspricht.

7. Verfahren nach Anspruch 6, wobei die Informationen in Bezug auf den Zustand des Benutzers eine Herzfrequenz umfassen.

8. Verfahren nach Anspruch 6, wobei die Informationen in Bezug auf die Messumgebung einen Standort der elektronischen Vorrichtung, eine Temperatur und/oder eine Luftfeuchtigkeit bei der Blutdruckmessung umfassen.

9. Verfahren nach Anspruch 6, wobei die Informationen in Bezug auf die Haltung des Benutzers einen Zustand, in dem der Blutdruck unter Verwendung der linken oder rechten Hand gemessen wird, einen stehenden Zustand, einen sitzenden Zustand, einen dynamischen Zustand, einen statischen Zustand, einen Schlafzustand, einen Ruhezustand, einen Trainingszustand und/oder einen Zustand nach dem Training umfassen.

## Revendications

1. Dispositif électronique (200) comprenant :
au moins un capteur (210 ; 220) incluant un capteur biométrique (210) ;
un processeur (250) ; et
une mémoire (230) connectée de manière opérationnelle au processeur (250),
où, lorsqu'elle est exécutée, la mémoire (230) stocke des instructions qui amènent le processeur (250) à :
obtenir des informations de l'au moins un capteur (210 ; 220) relatives à au moins un parmi un état d'un utilisateur, une posture de l'utilisateur ou un environnement de mesure, en réponse à la détection d'une partie du corps de l'utilisateur contactant ou approchant le capteur biométrique (210),
sélectionner un modèle de forme d'onde parmi une pluralité de modèles de forme d'onde stockés dans la mémoire, sur la base d'au moins l'un parmi l'état de l'utilisateur, la posture de l'utilisateur ou l'environnement de mesure,
mesurer une similitude en comparant un signal de photopléthysmogramme (PPG) obtenu de l'au moins un capteur (210 ; 220) et le modèle de forme d'onde sélectionné,
déterminer si la similitude du signal PPG s'inscrit dans une plage prédéfinie, sur la base de si le nombre de formes d'onde ayant des similitudes supérieures ou égales à une valeur seuil est supérieur ou égal à un nombre de référence de formes d'onde,
si la similitude du signal PPG s'inscrit dans la plage prédéfinie :
déterminer une valeur de pression artérielle de l'utilisateur en utilisant le modèle de forme d'onde sélectionné,
si la similitude du signal PPG ne s'inscrit pas dans la plage prédéfinie :
générer une forme d'onde représentative en utilisant le signal PPG,
mesurer la valeur de la pression artérielle de l'utilisateur sur la base de la forme d'onde représentative générée, et
ajouter un modèle de forme d'onde correspondant à la forme d'onde représentative générée dans la mémoire.

2. Dispositif électronique (200) selon la revendication 1, où l'au moins un capteur (210 ; 220) comprend :
le capteur biométrique (210) configuré pour obtenir les informations relatives à l'état de l'utilisateur ; et
un capteur supplémentaire (220) configuré pour obtenir les informations relatives à au moins l'un parmi la posture de l'utilisateur ou l'environnement de mesure,
où le capteur supplémentaire (220) comprend un capteur de gestes, un capteur gyroscopique (222), un capteur de pression atmosphérique, un capteur magnétique, un capteur d'accélération (221), un capteur de préhension, un capteur de proximité (223), un capteur de couleur, un capteur infrarouge (IR), un capteur de température, un capteur d'humidité (225), ou un capteur d'éclairement.

3. Dispositif électronique (200) selon la revendication 1, où les informations relatives à l'état de l'utilisateur comprennent une fréquence cardiaque.

4. Dispositif électronique (200) selon la revendication 1, où les informations relatives à l'environnement de mesure comprennent au moins un parmi un emplacement du dispositif électronique, une température ou une humidité lorsque la pression artérielle est mesurée.

5. Dispositif électronique (200) selon la revendication 1, où les instructions comprennent une instruction qui amène le processeur (250) à :
générer la forme d'onde représentative sur la base d'au moins l'un parmi l'état de l'utilisateur, la posture de l'utilisateur ou l'environnement de mesure lors de l'ajout du modèle de forme d'onde correspondant à la forme d'onde représentative, et
ajouter la forme d'onde représentative générée au modèle de forme d'onde.

6. Procédé de fonctionnement d'un dispositif électronique, le procédé comprenant :
obtenir des informations d'au moins un capteur incluant un capteur biométrique relatives à au moins un parmi un état d'un utilisateur, une posture de l'utilisateur ou un environnement de mesure, en réponse à la détection d'une partie du corps de l'utilisateur contactant ou approchant le capteur biométrique (210) ;
sélectionner un modèle de forme d'onde parmi une pluralité de modèles de forme d'onde stockés dans le dispositif électronique, sur la base des informations obtenues ;
mesurer une similitude en comparant un signal de photopléthysmogramme (PPG) obtenu de l'au moins un capteur et le modèle de forme d'onde sélectionné ;
déterminer si la similitude du signal PPG s'inscrit dans une plage prédéfinie, sur la base de si le nombre de formes d'onde ayant des similitudes supérieures ou égales à une valeur seuil est supérieur ou égal à un nombre de référence de formes d'onde ;
si la similitude du signal PPG s'inscrit dans la plage prédéfinie :
déterminer une valeur de pression artérielle de l'utilisateur en utilisant le modèle de forme d'onde sélectionné et un signal biométrique mesuré ;
si la similitude du signal PPG ne s'inscrit pas dans la plage prédéfinie :
générer une forme d'onde représentative en utilisant le signal PPG,
mesurer la valeur de la pression artérielle de l'utilisateur sur la base de la forme d'onde représentative générée, et
ajouter un modèle de forme d'onde correspondant à la forme d'onde représentative générée dans la mémoire.

7. Procédé selon la revendication 6, où les informations relatives à l'état de l'utilisateur comprennent une fréquence cardiaque.

8. Procédé selon la revendication 6, où les informations relatives à l'environnement de mesure comprennent au moins un parmi un emplacement du dispositif électronique, une température ou une humidité lorsque la pression artérielle est mesurée.

9. Procédé selon la revendication 6, où les informations relatives à la posture de l'utilisateur comprennent au moins un parmi un état où la pression artérielle est mesurée en utilisant la main gauche ou droite, un état debout, un état assis, un état dynamique, un état statique, un état de sommeil, un état de repos, un état d'exercice ou un état après l'exercice.
